# EUROPEAN PATENT APPLICATION

(11) **EP 2 698 115 A1**
(43) Date of publication of application: **19.02.2014**
(21) Application number: 12180657.4
(22) Date of filing: 16.08.2012
(51) Int. Cl.: A61B 10/02, A61B 17/16

(54) **Biopsy device**

(71) Applicant: AprioMed AB, 754 50 UPPSALA (SE)
(72) Inventor: Spengler, Håkan, 757 57 UPPSALA (SE)
(74) Representative: Brann AB

(57) **Abstract**

The present invention relates to a biopsy device (1) for obtaining a tissue sample. The biopsy device comprises an elongated tube shaped drill (2), extending along a longitudinal axis A, and comprising a cutting edge arrangement (3) at a distal end (4) of said drill (2). The drill (2) further comprises at least one flute (5) extending helically, along said longitudinal axis A, at an outer surface (6) of said drill (2), wherein said flute (5) extends from said distal end (4) and along a substantial part (7) of said outer surface (6) of said drill (2). The drill (2) is provided with an opening (10) at its distal end (4), wherein said opening (10) being in communication with a cavity (16) adapted to receive said tissue sample. The cutting edge arrangement (3) comprises a first cutting edge (11), adapted to perform a cutting action when said drill (2) is rotated in a first direction (d₁), and said first cutting edge (11) comprises at least one longitudinal cutting edge (13) extending essentially along said longitudinal axis A.

## Description

### Field of the invention

The present invention relates to a biopsy device, and in particular to a biopsy device for obtaining a tissue sample from a bone or a sclerotic lesion, according to the preamble of the independent claim.

### Background of the invention

Biopsy sampling in bone or sclerotic lesions may be difficult to carry out with the aid of a biopsy needle, e.g. in bone the lesion is often delimited by the hard surface layer of the bone, i.e. the cortical bone tissue. Today there are several known methods to gain access to the lesion in the bone, and one way is to introduce a conventional spiral drill equipped with a cannula, drill through the cortical bone, and then remove the drill. Thereafter, a biopsy needle may be introduced through the cannula to obtain a biopsy sample. A drawback with this method is that the cannula can not be inserted through the drilled hole because the outer diameter of the cannula is larger than the outer diameter of the drill, therefore the cannula may easily be moved out of position making it difficult to find the drilled hole to introduce the biopsy needle.

Another method of accessing hard tissue is known from US 5423824 A, which discloses a device for puncturing cortical bone. The device consists of a cannula and inside of it an axially movable needle. The distal end of the needle has an excentrically shaped tip, which by drilling forms a hole, larger than the outer diameter of the needle, through corticalis, whereupon, the enclosing cannula can be inserted into the drilled hole for securing the cannula's position. The sample is obtained by removing the drill and introducing appropriate biopsy needle or by simply using the discharge of chips from the drill when entering the drill into the bone, as tissue sample. However, when using the discharge of chips from the drill it may be difficult to obtain a sufficient amount of tissue sample from the bone.

In US 4696308 A an apparatus for obtaining a core sample including an elongate guide pin and a hollow drill element, is shown. The drill element has an open front end provided with a cutting edge and an open rear end provided with an axial engagement structure for engaging an associated driving tool. A longitudinal aperture extends from the front end of the drill element to the rear end with the aperture having a smooth uninterrupted interior periphery throughout at least a major portion of its length. A core of the hard tissue is received in the longitudinal aperture.

In US 4306570 A an universally applicable biopsy needle including two counter-rotating tubes having oppositely facing sawteeth formed on the distal ends thereof, is shown.

US6315737 B1 shows a biopsy needle with a conduit for receiving a hard biopsy sample and a fluid biopsy sample. The conduit has a first cross-sectional area. The biopsy needle has an end with a penetrating bit operable for coring the hard biopsy sample. The end has a second cross-sectional area less than the first cross-sectional area. Therefore, the hard biopsy sample has a cross-sectional area substantially equal to the second cross-sectional area. A space is thus left between the hard biopsy sample and the conduit when the hard biopsy sample is received therein. The biopsy needle has a retainer located at an interior of the conduit for holding the cored hard biopsy sample within the conduit.

The inventors of the present invention has identified a need for an improved biopsy device, which provides for a simplified procedure for obtaining a tissue sample, and which may be used in hard and soft tissue.

An object of the present invention is to provide a biopsy device which forms a hole which is sufficiently large to obtain an adequate amount of tissue sample, and which at the same time ensures that the biopsy device remains in its position until the sample has been obtained.

A further object of the present invention is to provide a biopsy device which is suitable for obtaining a sample in both soft and hard tissue.

Yet another obj ect is to provide a biopsy device which provides an improved procedure for obtaining a sample and which serves for a less painful procedure for the patient.

### Summary of the invention

The above-mentioned objects are achieved by the present invention according to the independent claim.

Preferred embodiments are set forth in the dependent claims.

In accordance with the present invention the biopsy device, for obtaining a tissue sample, comprises an elongated tube shaped drill, extending along a longitudinal axis A, and comprising a cutting edge arrangement at a distal end of said drill. The drill further comprises at least one flute extending helically, along said longitudinal axis A, at an outer surface of said drill. The flute extends from said distal end and along a substantial part of said outer surface of said drill. The drill is provided with an opening at its distal end, wherein said opening being in communication with a cavity adapted to receive said tissue sample. The cutting edge arrangement comprises a first cutting edge, adapted to perform a cutting action when said drill is rotated in a first direction, and said first cutting edge comprises at least one longitudinal cutting edge extending essentially along said longitudinal axis A.

### Short description of the appended drawings

Figure 1 shows a side view of the bone biopsy device according to one embodiment of the present invention.
Figure 2 shows an elevated view of the bone biopsy device according to one embodiment of the present invention.
Figure 3 shows a distal portion of the elongated drill, the drill comprises a first cutting edge and a second cutting edge according to one embodiment of the present invention.
Figure 4 shows a side view of the bone biopsy device according to one embodiment of the present invention.
Figure 5 shows the first cutting edge and the second cutting edge of the elongated tube shaped drill, the first cutting edge includes a radial cutting edge, an inclined cutting edge and an longitudinal cutting edge, according to one embodiment of the present invention.
Figure 6 shows a longitudinal cross-sectional view of the biopsy device comprising a tapering distal end according to one embodiment of the present invention.
Figure 7 shows a longitudinal cross-sectional view of the biopsy device provided with a decreasing inner diameter, according to one embodiment of the present invention.
Figure 8 shows a longitudinal cross-sectional view of the biopsy device with a cutting edge arrangement comprising an inclined cutting edge, according to one embodiment of the present invention.

### Detailed description of preferred embodiments of the invention

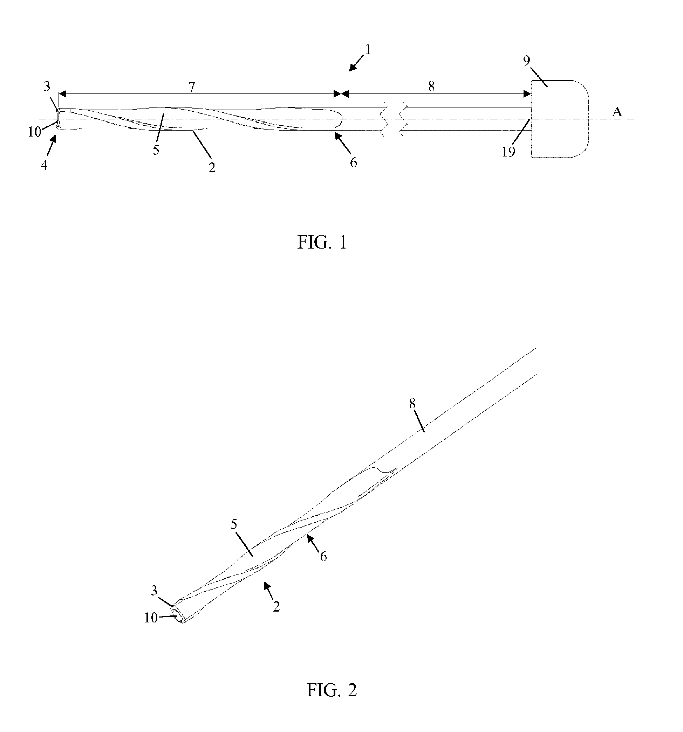

Figure 1 shows a biopsy device 1, for obtaining a tissue sample, the biopsy device 1 comprising an elongated tube shaped drill 2, extending along a longitudinal axis A, and comprising a cutting edge arrangement 3 at a distal end 4 of the drill 2. The drill 2 comprises at least one flute 5 extending helically, along the longitudinal axis A, at an outer surface 6 of the drill 2, wherein the flute 5 extends from the distal end 4 and along a substantial part 7 of the outer surface 6 of the drill 2. In the embodiment shown in Figure 1, the biopsy device 1 further comprises a shank portion 8 and a handle 9 arranged at a proximal end 19 of the elongated drill 2. Preferably, the length of the substantial part 7 is between 5-30 mm. The length of the shank portion 8 is preferably 30-200 mm. Preferably, the at least one flute 5 extends between the distal end 4 and the shank portion 8. The handle 9 facilitates rotation of the drill 2 during insertion.

Figure 2 illustrates that the elongated tube shaped drill 2 is provided with an opening 10 at its distal end 4. The opening 10 is in communication with a cavity (not shown in Figure 2) adapted to receive the tissue sample. The cavity 16 extends within and at least partly along the length of the elongated tube shaped drill 2.

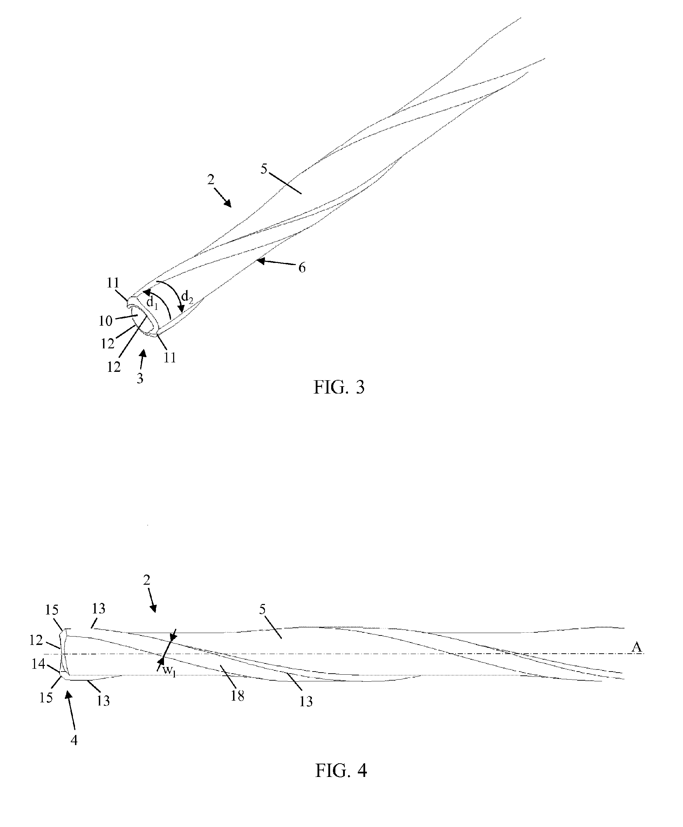

According to the embodiment illustrated in Figure 3, the cutting edge arrangement 3 comprises a first cutting edge 11, adapted to perform a cutting action when the drill 2 is rotated in a first direction d₁, and a second cutting edge 12, adapted to perform a cutting action when the drill 2 is rotated in a second direction d₂, opposite the first direction d₁. The cutting edges 11, 12 are preferably symmetrically arranged, such that the drill 2 is concentric. The shape of the first cutting edge 11 is advantageous when introducing the biopsy device 1 in bone or sclerotic lesions, i.e. in relatively hard tissue. Consequently, when introducing the biopsy device 1 in bone or sclerotic lesions the drill is preferably rotated in the first direction d₁. The shape of the second cutting edge 12 is advantageous when introducing the biopsy device 1 in relatively soft tissue. Therefore, when introducing the biopsy device 1 in soft tissue the drill 2 is preferably rotated in the second direction d₂. Preferably, the first direction d₁ is clockwise direction, and the second direction d₂ is counter-clockwise direction. However, the cutting edge arrangement 3 may be laterally reversed, wherein the first direction d₁ is counter-clockwise direction and the second direction d₂ is clockwise direction.

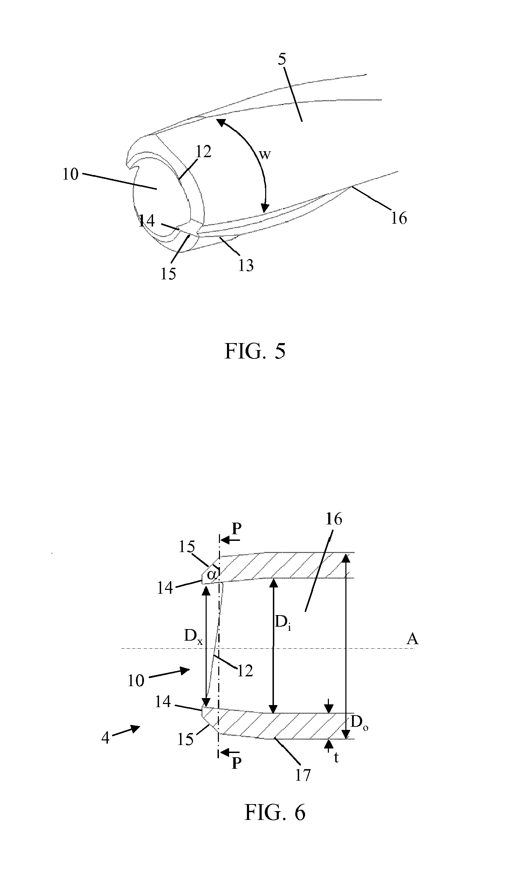

According to one embodiment, as illustrated in Figure 4, the first cutting edge 11 comprises at least one longitudinal cutting edge 13 extending essentially along the longitudinal axis A. Preferably, the longitudinal cutting edge 13 extends between the distal end 4 and the shank portion 8 of the drill 2. Furthermore, the first cutting edge 11 comprises at least one radial cutting edge 14 essentially extending along a radius of the drill 2, the radial cutting edge 14 being arranged at the distal end 4 of the drill 2. Furthermore, the first cutting edge 11 comprises at least one inclined cutting edge 15 essentially extending at an angle α (see Figure 6) with respect to a plane P perpendicular to the longitudinal axis A, the inclined cutting edge 15 being arranged at the distal end 4 of the drill 2.

Preferably, the number of longitudinal cutting edges 13 is between 2-4. According to one embodiment, each longitudinal cutting edge 13 is arranged at and extends along a longitudinal cutting edge portion 18. As seen in Figure 4, the longitudinal cutting edge portion 18 is elevated with respect to the flute 5, and between adjacent longitudinal cutting edge portions 18 a flute 5 is provided. The width w₁ of the longitudinal cutting edge portion 18 is preferably between 0,2-0,8 mm. The number of flutes 5 is preferably 2-4. According to one embodiment, the pitch angle of the flute 5 is 5-30 degrees.

In one embodiment, as illustrated in Figure 5, the longitudinal cutting edge 13 is interconnected with the inclined cutting edge 15. The longitudinal cutting edge 13 extends helically along the drill 2, at the outer surface 6 of the drill 2. The flute 5 has a width w extending along the circumference of the drill 2. Preferably, the width w is 1-6 mm.

According to one embodiment, as illustrated in Figure 6, the second cutting edge 12 is provided at the distal end 4 of the drill 2, and has an extension essentially in a plane P perpendicular to the longitudinal axis A, and along a circumference of the drill 2. Figure 6 further shows that a cavity 16 is provided within the elongated tubular drill 2. The cavity 16 is adapted to receive a tissue sample 10, and the opening 10 is in communication with the cavity 16. The inclined cutting edge 15 essentially extends at an angle α with respect to the plane P perpendicular to the longitudinal axis A. Preferably, the angle α is approximately 30-60 degrees.

In one embodiment, as seen in Figure 6, the cavity 16 has a diameter Dᵢ, and preferably, the diameter Dᵢ of the cavity 16 is between 1-4 mm. The elongated tubular drill 2 further comprises a wall 17 enclosing the cavity 16. In one embodiment, the wall 17 of the elongated tubular drill 2 has a thickness t of approximately 0,4-0,8 mm. The outer diameter Dₒ of the drill 2 is preferably between 1,5-5,0 mm.

According to one embodiment, as illustrated in Figure 6, the inner diameter Dᵢ is decreasing towards and close to the distal end 4. Preferably, the difference between the inner diameter Dᵢ and a diameter Dₓ at the distal end 4 is approximately 0,5-3 mm. A decreasing inner diameter Dᵢ reduces the friction when the sample enters into the cavity 6. The decreasing inner diameter Dᵢ is preferably achieved by gun drilling or by means of swaging. In the embodiment shown in Figure 6, the outer diameter Dₒ of the drill 2 is decreasing towards and close to the distal end 4. The decreasing outer diameter Dₒ may be achieved by swaging.

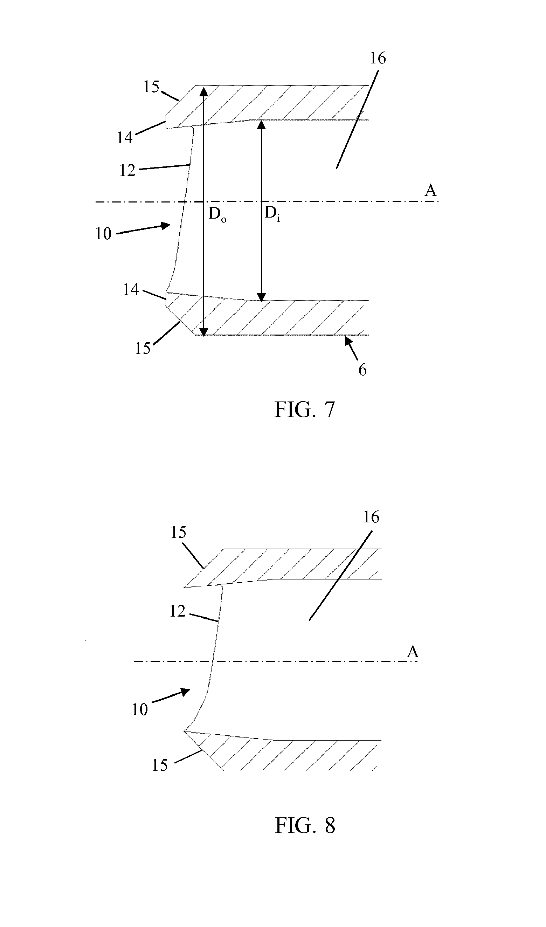

According to the embodiment shown in Figure 7, the drill 2 has a decreasing inner diameter Dᵢ, however, the outer diameter Dₒ is unchanged at the distal end 4.

Figure 8 shows a longitudinal cross-sectional view of the biopsy device 1 according to one embodiment of the present invention. The first cutting edge 11 comprises an inclined cutting edge 15 and no radial cutting edge 14. An inclined cutting edge 15 and no radial cutting edge 14 is advantageous in that the length of the inclined cutting edge 15 is greater compared to a biopsy device 1 being provided with an inclined cutting edge 15 and a radial cutting edge 14, which provides for improved cutting properties. However, a biopsy device 1 comprising an inclined cutting edge 15 and a radial cutting edge 14 provides improved strength.

The present invention is not limited to the above-described preferred embodiments. Various alternatives, modifications and equivalents may be used. Therefore, the above embodiments should not be taken as limiting the scope of the invention, which is defined by the appending claims.

## Claims

1. Biopsy device (1), for obtaining a tissue sample, said biopsy device (1) comprising an elongated tube shaped drill (2), extending along a longitudinal axis A, and comprising a cutting edge arrangement (3) at a distal end (4) of said drill (2);
said drill (2) comprising at least one flute (5) extending helically, along said longitudinal axis A, at an outer surface (6) of said drill (2), wherein said flute (5) extends from said distal end (4) and along a substantial part of said outer surface (6) of said drill (2), **characterized in that** said drill (2) is provided with an opening (10) at its distal end (4), wherein said opening (10) being in communication with a cavity (16) adapted to receive said tissue sample, and **in that** said cutting edge arrangement (3) comprises a first cutting edge (11), adapted to perform a cutting action when said drill (2) is rotated in a first direction (d₁), and **in that** said first cutting edge (11) comprises at least one longitudinal cutting edge (13) extending essentially along said longitudinal axis A.

2. Biopsy device according to claim 1, wherein said cutting edge arrangement (3) comprises a second cutting edge (12), adapted to perform a cutting action when said drill (2) is rotated in a second direction (d₂), opposite said first direction (d₁).

3. Biopsy device according to any of claims 1-2, wherein said first cutting edge (11) comprises at least one radial cutting edge (14) essentially extending along a radius of said drill (2), said radial cutting edge (14) being arranged at said distal end (4) of said drill (2).

4. Biopsy device according to any of claims 1-3, wherein said first cutting edge (11) comprises at least one inclined cutting edge (15) essentially extending at an angle α with respect to a plane P perpendicular to said longitudinal axis A, said inclined cutting edge (15) being arranged at said distal end (4) of said drill (2).

5. Biopsy device according to any of claims 1-4, wherein said second cutting edge (12) is provided at said distal end (4) of said drill (2), and has an extension essentially in said plane P perpendicular to said longitudinal axis A, and along a circumference of said drill (2).

6. Biopsy device according to any of claims 1-5, wherein said longitudinal cutting edge (13) extends helically along said drill (2), at said outer surface (6) of said drill (2).

7. Biopsy device according to any of claims 4-6, wherein said longitudinal cutting edge (13) is interconnected with said inclined cutting edge (15).

8. Biopsy device according to any of claims 1-7, wherein each longitudinal cutting edge (13) is arranged at and extends along a longitudinal cutting edge portion (18).

9. Biopsy device according to any of claims 1-8, wherein said cutting edges (11, 12) are symmetrically arranged.

10. Biopsy device according to any the preceding claims, wherein said first direction (d₁) is clockwise direction.

11. Biopsy device according to any of the preceding claims, wherein said second direction (d₂) is counter-clockwise direction.

12. Biopsy device according to any of the preceding claims, wherein a pitch angle of said flute (5) is approximately 5-30 degrees.

13. Biopsy device according to any of the preceding claims, wherein said flute (5) has a width w extending along the circumference of said drill (2), wherein said width w is preferably 1-6 mm.

14. Biopsy device according to any of the preceding claims, wherein said cavity (16) has a diameter (Dᵢ), being approximately 1-4 mm.

15. Biopsy device according to claim 14, wherein said diameter (Dᵢ) of said cavity (16) is decreasing towards and close to said distal end (14).
